# EUROPEAN PATENT SPECIFICATION

(11) **EP 3 845 224 B1**
(45) Date of publication and mention of the grant of the patent: **06.11.2024**
(21) Application number: 19855442.0
(22) Date of filing: 30.08.2019
(51) Int. Cl.: A61K 9/70, A61K 31/27, A61K 47/02, A61K 47/24, A61K 45/06, A61K 47/22

(54) **RIVASTIGMINE PATCH FOR LONG-TERM ADMINISTRATION**
RIVASTIGMIN-PATCH ZUR LANGZEITVERABREICHUNG
TIMBRE DE RIVASTIGMINE POUR ADMINISTRATION DE LONGUE DURÉE

(30) Priority: 31.08.2018 KR 20180103855
(43) Date of publication of application: 07.07.2021
(73) Proprietor: SK Chemicals Co., Ltd., Seongnam-si, Gyeonggi-do 13494 (KR)
(72) Inventor: KIM, Namho, Seongnam-Si, Gyeonggi-do 13494 (KR); CHOI, Wonjae, Seongnam-Si, Gyeonggi-do 13494 (KR); YOO, Hunseung, Seongnam-Si, Gyeonggi-do 13494 (KR); LEE, Soo Min, Seongnam-Si, Gyeonggi-do 13494 (KR); PARK, Yeo-Jin, Seongnam-Si, Gyeonggi-do 13494 (KR); KIM, Sung-Hyuk, Seongnam-Si, Gyeonggi-do 13494 (KR); YOO, Jong-su, Seongnam-Si, Gyeonggi-do 13494 (KR); LEE, Young-me, Seongnam-Si, Gyeonggi-do 13494 (KR)
(74) Representative: Cabinet Nony
(86) International application number: PCT/KR2019/011211
(87) International publication number: WO 2020/046072

(56) References cited:
- WO-A1-2016/018858
- WO-A2-2014/111790
- JP-A- 2006 513 160
- JP-A- H06 199 660
- KR-A- 20050 037 405
- KR-A- 20120 130 073
- KR-A- 20160 005 024
- US-A1- 2017 112 781
- US-A1- 2018 028 663

## Description

### [Technical Field]

The present invention relates to a patch comprising rivastigmine or a pharmaceutically acceptable salt thereof as an active ingredient, which can be continuously applied for a long period of time, preferably 3 days or more, more preferably 4 days or more.

### [Background Art]

Rivastigmine is used to treat or ameliorate Alzheimer's disease, etc., and inhibit acetylcholinesterase in the central nervous system. Such rivastigmine is commercially available as a patch formulation.

U.S. Patent No. 6,335,031 relates to a transdermal composition containing rivastigmine or a salt thereof, and is characterized by using a stabilizer. Said invention discloses that it is difficult to secure a storage period necessary for commercial distribution since the transdermal composition containing rivastigmine is decomposed by oxidation reaction with oxygen even under closed storage conditions. In order to solve this problem, said invention discloses a composition for transdermal administration of rivastigmine wherein the composition comprises an antioxidant such as tocopherol, ascorbic acid, butylhydroxytoluene, butylhydroxyanisole, and propyl gallate.

US 2017/112781 A1 discloses a transdermal drug delivery system comprising rivastigmine.

To date, commercially available rivastigmine patches are only available for continuous administration over 24 hours. However, a patch that can be applied for a longer period of time is required for the convenience of the patient and the characteristics of the dementia patient to be treated. In order to release rivastigmine at an appropriate release rate over an extended time interval of more than 24 hours, long-term administration patches are bound to contain large amounts of rivastigmine, and due to this high content, a variety of problems, including cold flow problem arise.

That is, there is a need for a patch having good physical and chemical stability while releasing rivastigmine at an appropriate release rate for a long period of time even if it contains a high content of rivastigmine or a salt thereof.

### [Disclosure]

### [Technical Problem]

Therefore, the problem to be solved by the present invention is to provide a patch which is physically stable and can release rivastigmine at an appropriate release rate for a long period of time and in which the stability of rivastigmine also are secured, even if the patch contains a high content of rivastigmine or a pharmaceutically acceptable salt thereof for long-term therapeutic application.

### [Technical Solution]

In order to solve the above problem, the present disclosure provides a rivastigmine patch for transdermal treatment comprising (1) a backing film that serves to protect the patch and is inert to the components of the matrix, (2) a rivastigmine matrix layer that comprises rivastigmine or a pharmaceutically acceptable salt thereof, (3) a self-adhesive matrix layer that comprises an adhesive that adheres to the skin, and (4) a release liner to be removed before use, wherein the content per patch area of rivastigmine or a pharmaceutically acceptable salt thereof is 2 to 15 mg/cm², preferably 3.6 to 10.8 mg/cm², more preferably 5.4 to 9 mg/cm² based on the rivastigmine free base, and the rivastigmine matrix layer comprises at least one selected from the group consisting of silicate, microcrystalline cellulose, and crospovidone.

In order to prepare a patch that is absorbed into the skin by releasing rivastigmine for a long period of time, preferably at least 2 days, more preferably 3 days or more, even more preferably 4 days or more, there is no choice but to increase the content of rivastigmine. For this reason, the height (thickness) of the rivastigmine matrix layer must exceed a certain level. In this case, the stability of the patch during storage or adhesion to the skin, particularly the stability of the layer comprising rivastigmine, became a problem, and the release rate of rivastigmine was also greatly affected.

While studying various means, the present inventors completed the present invention by confirming that physical and chemical stability can be secured through the same method as the present disclosure and that an appropriate release rate can be maintained even during long-term application.

The rivastigmine patch of the present disclosure comprises (1) a backing film, (2) a rivastigmine matrix layer comprising rivastigmine or a pharmaceutically acceptable salt thereof, (3) a self-adhesive matrix layer comprising an adhesive that adheres to the skin, and (4) a release liner, which will be described in detail below.

### Rivastigmine matrix layer

In the present invention, the rivastigmine matrix layer has a content per patch area of rivastigmine or a pharmaceutically acceptable salt thereof of 2 to 15 mg/cm², preferably 3.6 to 10.8 mg/cm², more preferably 5.4 to 9 mg/cm² based on the rivastigmine free base, and the rivastigmine matrix layer comprises at least one selected from the group consisting of silicate, microcrystalline cellulose and crospovidone in order to achieve the above object.

The aforementioned physical and chemical stability can be ensured through the above-mentioned component(s). In addition, due to the characteristics of rivastigmine, it is mixed in the adhesive layer to provide adhesion, and when rivastigmine is lost through skin penetration, the adhesion force may be reduced. In this case, it is believed that the specific components used in the present invention compensate for the loss of adhesive force and prevent structural deformation between the backing film and other layers. However, the present invention is not limited to these theoretical ideas.

In the present invention, as the silicate, magnesium aluminometasilicate (e.g., Neusilin^{™}), calcium silicate (e.g., Florite R^{™}), or a mixture thereof may be used as the silicate. It is preferable to use a powdery product rather than a granular product as the silicate product. For example, among the Neusilin products, UFL2^{™} in powder form is more preferable than US2^{™} in granular form. It is easy to make the surface homogeneous when manufacturing a patch only when using a powdery product.

Preferably, in one embodiment of the present invention, the rivastigmine matrix layer comprises 10-50% by weight of rivastigmine, 10-30% by weight of microcrystalline cellulose, and the residual quantity of the acrylic adhesive based on the total weight of the rivastigmine matrix layer.

Preferably, in one embodiment of the present invention, the rivastigmine matrix layer comprises 10-50% by weight of rivastigmine, 10-30% by weight of magnesium aluminometasilicate, and the residual quantity of the acrylic adhesive based on the total weight of the rivastigmine matrix layer.

Preferably, in one embodiment of the present invention, the rivastigmine matrix layer comprises 10-50% by weight of rivastigmine, 5-25% by weight of crospovidone, and the residual quantity of the acrylic adhesive based on the total weight of the rivastigmine matrix layer.

Preferably, in one embodiment of the present invention, the rivastigmine matrix layer comprises 10-50% by weight of rivastigmine, 2-15% by weight of calcium silicate, and the residual quantity of the acrylic adhesive based on the total weight of the rivastigmine matrix layer.

The rivastigmine matrix layer may optionally further comprises an antioxidant of 0.01 to 1% by weight, preferably 0.05 to 0.3% by weight. In this case, the content of the acrylic adhesive decreases as the antioxidant is added.

As the acrylic adhesive for forming the rivastigmine matrix layer, a copolymer of a (meth)acrylic acid ester monomer having an alkyl group of 1 to 12 carbon atoms and a polar monomer being copolymerizable with the (meth)acrylic acid ester monomer may be used. Examples of the (meth)acrylic acid ester monomer include butyl (meth)acrylate, hexyl (meth)acrylate, n-octyl (meth)acrylate, isooctyl (meth)acrylate, and 2-ethylhexyl (meth)acrylate, isononyl (meth)acrylate, and the like. In addition, examples of the polar monomer copolymerizable with the (meth)acrylic acid ester-based monomer include monomers containing carboxyl groups such as (meth)acrylic acid, maleic acid, and fumaric acid, and nitrogen-containing monomers like acrylamide, N-vinylpyrrolidone, and N-vinylcaprolactam. In the acrylic adhesive, the ratio of the (meth)acrylic acid ester monomer and the polar monomer is not particularly limited, but may generally have a weight ratio of 99 to 80: 1 to 20. Examples of such acrylic adhesives include DURO-TAK^{™} 87-202A, 387-2510, 87-2510, 87-4287, 387-2287, 87-2287, 387-2516, 87-2516, 387-2525, 87-2525, 87-2979, 87-2074, 87-235A, 87-2353, 387-2353, 87-2852, 87-2051, 387-2051, 87-2052, 387-2052, 387-2054, 87-2054, 87-2677, 87-2194, 387-2196, 387-2825, 87-2825, 87-502A, 87- 503A, 87-504A, 87-9088, 87-4098, and the like. Preferably, a random copolymer of 58-66 parts by weight of 2-ethylhexyl acrylate, 28-36 parts by weight of methyl acrylate, and 4-8 parts by weight of acrylic acid (e.g., Duro-Tak^{®} 87-235A) can be used as the acrylic adhesive in terms of miscibility and compatibility.

The rivastigmine matrix layer of the present invention may further comprises an acrylic polymer like a copolymer of butyl methacrylate and methyl methacrylate (preferably, a copolymer having a weight average molecular weight of 130,000-170,000 g/mol, for example, PLASTOID^{™} B, EVONIK), methacrylic acid copolymer type A, B, C (preferably, methacrylic acid copolymer, type A, for example, Eudragit L100), and the like.

The rivastigmine matrix layer may further comprise an antioxidant to secure the chemical stability of rivastigmine or a pharmaceutically acceptable salt thereof, and such antioxidants, preferably, tocopherol, butylated hydroxyanisole (BHA), butylated hydroxytoluene (BHT) or a mixture thereof may be used. More preferably, tocopherol is used as an antioxidant in the patch of the present invention.

### Self-adhesive matrix layer

The rivastigmine patch of the present invention comprises a self-adhesive matrix layer in contact with skin, and a previously known skin adhesive layer may be used as the skin-contact self-adhesive matrix layer.

The self-adhesive matrix layer may comprise any one or more selected from an acrylic adhesive, a rubber-based adhesive, and a silicone-based adhesive as an adhesive polymer (adhesive).

As the acrylic adhesive, examples mentioned in the rivastigmine matrix layer may be used. As the silicone adhesive, preferably, dimethylsiloxane-treated trimethylated silica may be used. For example, DOW CORNING's BIO-PSA^{™} 7-4101, 7-4201, 7-4301, 7-4102, 7-4202, 7-4302, 7-4103, 7-4203, 7-4303, 7-4401, 7-4501, 7-4601, 7-4402, 7-4502, 7-4602, 7-4403, 7-4503, 7-4603, or mixtures thereof, may be used.

In the system of the present invention, it is preferable to use a mixture of two or more types of silicone adhesives having different physical properties as the silicone-based adhesive. Particularly, the mixture of BIO-PSA 7-4201 and 7-4301 (more preferably, a 1:1 weight ratio mixture) was more excellent in improving adhesion in patches comprising a large amount of rivastigmine.

As a rubber-based adhesive, for example, an adhesive formed by mixing a high-molecular and low-molecular polybutylene (PB) or poly isobutylene (PIB) may be used. Alternatively, an adhesive formed by mixing a styrene block copolymer-based styrene-isoprene-styrene (SIS) and polyisobutylene (PIB) may be used.

In this self-adhesive matrix layer, in order to aid in the stability of rivastigmine of the rivastigmine matrix layer in contact, the aforementioned antioxidant may be comprises.

In the self-adhesive matrix layer, an appropriate penetration enhancer may be further comprised in order to aid in the absorption of rivastigmine of the rivastigmine matrix layer in contact.

In addition, in the self-adhesive matrix layer, an appropriate adhesion enhancing agent may be further comprised in order to enhance the adhesion of the rivastigmine matrix layer in contact.

### Release-controlling membrane

Preferably, the rivastigmine patch of the present invention may comprise a release-controlling membrane between the rivastigmine matrix layer and the self-adhesive matrix layer, and such release-controlling membrane is, for example, polypropylene membrane (e.g., Celgard^{™} 2400), polyethylene membrane (e.g., CoTran^{™} 9719 or 9720), or polyethylene membrane with a vinyl acetate ratio of 4.5-19% (e.g., CoTran^{™} 9707, 9702, 9728) can be used. The release-controlling membrane according to the present invention may have a porosity of up to 90% (e.g., Solupor^{™} 10P05A, Celgard^{™} 2400).

An ethylene vinyl acetate membrane is preferable as such a release-controlling membrane, and for example, 3M CoTran^{™} 9705, 9707, etc. may be preferably used as such an ethylene vinyl acetate membrane.

Typically, the release-controlling membrane has a thickness of 0.01 to 0.15 mm. The preferred thickness of the membrane is 0.025 to 0.080 mm.

### Backing film and Release liner

The backing film is blocked, i.e., distal. In a preferred embodiment, this backing film may be polyolefin, in particular polyethylene, or polyester as well as polyurethane. Further, preferably, a polyester foil such as polyethylene terephthalate foil can be used.

The release liner is a pull-off layer that is removed immediately before use. For example, polyurethane, polyvinyl acetate, polyvinylidene chloride, polypropylene, polycarbonate, polystyrene, polyethylene, polyethylene terephthalate, and polybutylene terephthalate may be used as the release liner. Alternatively, a paper surface-coated with the above polymer(s) may be used. Preferably, one-sided siliconized polymer foil is used.

### [Advantageous Effects]

The present invention provides a rivastigmine patch that can be applied therapeutically for a long time by securing various aspects of physical and chemical stability, and releasing rivastigmine at a good rate for a long time, even if it comprises a high content of rivastigmine per unit area of the patch.

The rivastigmine patch of the present invention can freely control the release rate of rivastigmine by adjusting the release-controlling agent of the rivastigmine matrix layer or the self-adhesive matrix layer, or by adjusting the release controlling membrane, etc. Long-term stability is ensured even after controlling the release rate.

### [Brief Description of Drawings]

Figure 1 is a graph showing the cumulative permeation percentages of examples of the present invention and a commercially available Exelon^{™} patch in a permeation test.
Figure 2 is a graph showing the cumulative permeation amounts (µg/cm²) of examples of the present invention and a commercially available Exelon^{™} patch in a permeation test.

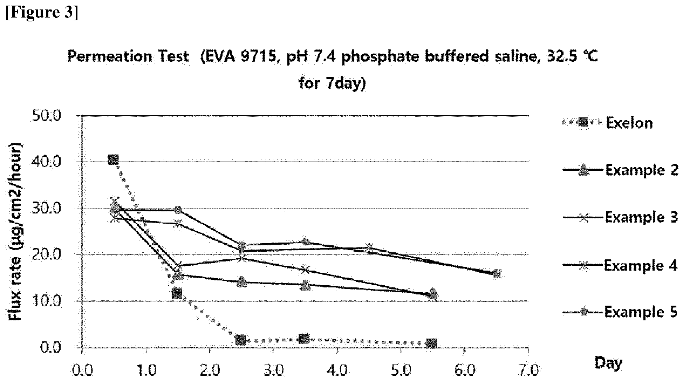

Figure 3 is a graph showing changes in permeation rate (µg/cm²/hour) of examples of the present invention and a commercially available Exelon^{™} patch in a permeation test.

### [Mode for Invention]

Hereinafter, the present disclosure is described in considerable detail with examples to help those skilled in the art understand the present disclosure. However, the following examples are offered by way of illustration and are not intended to limit the scope of the invention. It is apparent that various changes may be made without departing from the spirit and scope of the invention or sacrificing all its material advantages.

In the following comparative examples and examples, when expressed as simply "%" it means % by weight.

In the following comparative examples and examples, a PET film was used as a backing film, 3M's Scotchpak 9744 product was used as a release liner, and an ethylene vinyl acetate membrane, 3M CoTran^{™} 9707 was used as a release-controlling membrane.

In the following comparative examples and examples, unless otherwise stated, a self-adhesive polysiloxane polymer (Dow Coming@ BIO-PSA, 1:1 mixture of 7-4201 and 7-4301) containing 0.1% of DL-alpha-tocopherol was used as a self-adhesive matrix layer.

### Comparative Example 1 (4 day version of Exelon patch)

A patch consisting of (1) a backing film, (2) a self-adhesive matrix layer consisting of 30% by weight of rivastigmine, 20% by weight of plastoid B, 0.1% by weight of DL-alpha-tocopherol, and 49.9% by weight of a self-adhesive acrylic adhesive, (3) a self-adhesive matrix layer consisting of a self-adhesive polysiloxane polymer containing 0.1% by weight of DL-alpha-tocopherol, and (4) a release liner was prepared. It was prepared at 180 GSM (gram per square meter, g/m²) based on the drug layer (rivastigmine content corresponds to 7.2 mg/cm²; Exelon^{™} has 1.8 mg/cm²/day of rivastigmine content).

### Comparative Example 2 (4 day version without matrix support material)

A patch with double-layer system consisting of (1) a backing film, (2) a self-adhesive matrix layer consisting of 40% rivastigmine, 0.1% DL-alpha-tocopherol, and 59.9% self-adhesive acrylic adhesive, (3) a self-adhesive matrix layer consisting of a self-adhesive polysiloxane polymer containing 0.1% DL-alpha-tocopherol, and (4) a release liner was prepared. It was prepared at 180 GSM based on the drug layer (rivastigmine content corresponds to 7.2 mg/cm²).

### Example 1

A patch with a triple layer system consisting of (1) a backing film, (2) a self-adhesive matrix layer consisting of 40% rivastigmine, 20% microcrystalline cellulose, 0.1% DL-alpha-tocopherol, and 39.9% self-adhesive acrylic adhesive (Duro-Tak@ 87-235A), (3) a release-controlling membrane, (4) a self-adhesive matrix layer consisting of a self-adhesive polysiloxane polymer (Dow Coming@ BIO-PSA, 1:1 mixture of 7-4201 and 7-4301) containing 0.1% DL-alpha-tocopherol, and (5) a release liner was prepared. It was prepared at 180 GSM based on the drug layer.

### Example 2

A patch with a triple layer system consisting of (1) a backing film, (2) a self-adhesive matrix layer consisting of 40% rivastigmine, 20% Neusilin (UFL2^{™}), 0.1% DL-alpha-tocopherol, and 39.9% self-adhesive acrylic adhesive (Duro-Tak@ 87-235A), (3) a release controlling membrane, (4) a self-adhesive matrix layer consisting of a self-adhesive polysiloxane polymer (Dow Coming@ BIO-PSA, 1:1 mixture of 7-4201 and 7-4301) containing 0.1% DL-alpha-tocopherol and (5) a release liner was prepared. It was prepared at 180 GSM based on the drug layer.

### Example 3

A patch with a triple layer system consisting of (1) a backing film, (2) a self-adhesive matrix layer consisting of 40% rivastigmine, 15% crospovidone (Kollidone CL-M), 0.1% DL-alpha-tocopherol, and 44.9% self-adhesive acrylic adhesive (Duro-Tak@ 87-235A), (3) a release controlling membrane, (4) a self-adhesive matrix layer consisting of a self-adhesive polysiloxane polymer (Dow Coming@ BIO-PSA, 1:1 mixture of 7-4201 and 7-4301) containing 0.1% DL-alpha-tocopherol and (5) a release liner was prepared. It was prepared at 180 GSM based on the drug layer.

### Example 4

A patch with a triple layer system consisting of (1) a backing film, (2) a self-adhesive matrix layer consisting of 40% rivastigmine, 5% Florite R, 0.1% DL-alpha-tocopherol, and 54.9% self-adhesive acrylic adhesive (Duro-Tak@ 87-235A), (3) a release controlling membrane, (4) a self-adhesive matrix layer consisting of a self-adhesive polysiloxane polymer (Dow Corning^{®} BIO-PSA, 1:1 mixture of 7-4201 and 7-4301) containing 0.1% DL-alpha-tocopherol and (5) a release liner was prepared. It was prepared at 180 GSM based on the drug layer.

### Experimental Example 1 (Evaluation of Chemical Stability)

Stability evaluation of rivastigmine was performed while storing the comparative examples and examples in a room temperature environment and an accelerated environment (40 °C, 75%RH) in the following manner.

Ten round patches of 5 cm² size were placed in a 100 mL volumetric flask and diluted with an extraction solvent. After ultrasonic vibration for 60 minutes, 5 mL of this solution was taken at room temperature, put into a 10 mL volumetric flask, and evaporated for 30 minutes under a nitrogen stream. After putting the mobile phase, it was filtered through a 0.45 µm membrane filter.

### - Manufacturing of mobile phase

2.02 g of 1-heptanesulfonate sodium was added to a 1 L volumetric flask, diluted with purified water, and adjusted to pH 3.0 with dilute phosphoric acid. Then, it was filtered through a 0.45 µm membrane filter.

### - Preparation of extraction solvent

Methanol: ethyl acetate: triethylamine = 70: 30: 0.4 (V/V/V)

### - HPLC analysis conditions

Injection volume: 10 µL
Column: RP18 -C18, 250 mm X 4.6 mm, 5 µm
Detector: UV Detector (217 nm)
Flow rate: 1.0 mL / min
Run time: 30 minutes
Mobile phase: 10 mM Sodium-1-heptane sulphonate buffer: acetonitrile (72:28).

The results are shown in Table 1 below.

**[Table 1]**

| Total Impurities | Accelerated Environment 1 month | Long Term 3 months | Long Term 6 months |
|---|---|---|---|
| Comparative example 1 | 0.41% | 0.24% | 0.35% |
| Comparative example 2 | 0.45% | 0.30% | 0.41% |
| Example 1 | 0.50% | 0.31% | 0.44% |
| Example 2 | 0.39% | 0.19% | 0.31% |
| Example 3 | 0.36% | 0.20% | 0.47% |
| Example 4 | 0.58% | 0.22% | 0.33% |

As shown in Table 1, the rivastigmine patch according to the present invention maintained its stability even if it comprises a high amount of rivastigmine. However, comparatively, when Neusilin or Florite was comprised in the rivastigmine matrix layer, the stability was better than when other materials were used.

### Experimental Example 2 (Evaluation of Physical Stability)

The following physical stability was measured.

Shear measurement method: After forming a 5 cm² circular patch, static shear was measured by connecting a 200 g weight.

Probe Tack measurement method: After forming a 1.7 cm² circular patch, the probe tack was measured.

Cold flow measurement method: 5 cm² circular patch was formed. After pressing with a 1 kg weight for 1 week, the cold flow (creep test) was measured. Alternatively, after leaving it open at 60-80 °C for 1 week, the cold flow was measured.

The results are shown in Table 2.

**[Table 2]**

| | Shear (Attached area 5 cm², 200g) | Tack (g) | Cold flow (creep & heat test) |
|---|---|---|---|
| Comparative example 1 | Desorption within 24 hours | > 200 g | Very severe |
| Comparative example 2 | Desorption within 1 hour | 200 g | Very severe |
| Example 1 | 72 hours or more | 150∼200 g | Almost none |
| Example 2 | 72 hours or more | 100∼150 g | Almost none |
| Example 3 | 72 hours or more | 150∼200 g | Almost none |
| Example 4 | 72 hours or more | 100∼150 g | Almost none |

As shown in the results of Table 2, the patch according to the present invention showed excellent physical stability.

### Experimental Example 3 (Transdermal Penetration of Patch)

Transdermal penetration experiments using a commercially available Exelon@ patch were conducted under the following conditions.
Franz Diffusion Cell Experimental Parameters
Apparatus: Hansen Automated Franz diffusion cell sampling system
Cell volume: 7.0 mL
Receptor solution: solution phosphate buffered saline (PBS, 1.0 M)
Dose: 1.8 mg over a 1.0 cm2 area as rivastigmine base; occluded
Duration: 7 days
Temperature: 32.5 °C
Sample volume: 0.9 mL
Rinse volume: 1.6 mL
Sample analysis: HPLC
Membrane: 3M EVA 9715 membrane

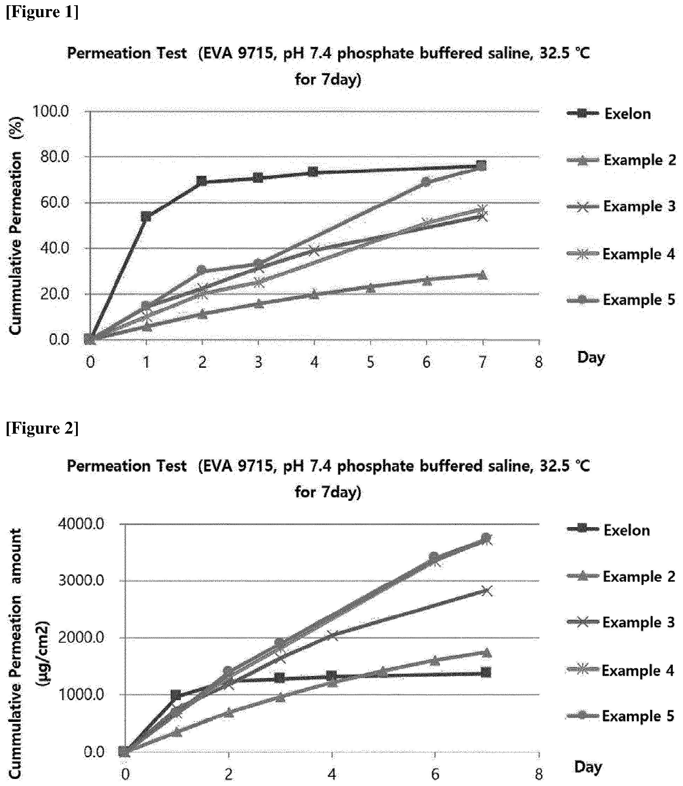

The results are shown in Tables 3 to 6 and Figures 1 to 2.

**[Table 3]**

| | Flux rate at 1 day (µg/cm²/h) | Flux rate at 4 day (µg/cm²/h) | Decreasing rate of penetration from 24 hours to 96 hours (%) |
|---|---|---|---|
| Exelon^{®} patch | 25∼30 µg | 0∼5 µg | 80∼90% |
| Examples 1∼4 | 26∼32 µg | 13∼23 µg | Within 15∼20% |

**[Table 4]**

| Day | Exelon^{®} patch | Example 1 | Example 2 | Example 3 | Example 4 |
|---|---|---|---|---|---|
| 0 | 0.0 | 0.0 | 0.0 | 0.0 | 0.0 |
| 1 | 53.6 | 5.7 | 14.4 | 10.0 | 14.4 |
| 2 | 68.9 | 11.2 | 22.5 | 20.0 | 29.9 |
| 3 | 70.6 | 15.7 | 31.3 | 25.0 | 33.1 |
| 4 | 72.9 | 19.7 | 38.9 | | |
| 5 | | 23.1 | | | |
| 6 | | 26.1 | | 51.0 | 68.6 |
| 7 | 76.0 | 28.4 | 54.0 | 57.0 | 75.4 |
| Content | 1.80 | 6.13 | 5.24 | 7.12 | 6.64 |

Table 4 shows the evaluation results of the cumulative penetration percentage (unit: %).

**[Table 5]**

| Day | Exelon^{®} patch | Example 1 | Example 2 | Example 3 | Example 4 |
|---|---|---|---|---|---|
| 1 | 965 | 350 | 757 | 670 | 680 |
| 2 | 1239 | 685 | 1179 | 1310 | 1390 |
| 3 | 1271 | 962 | 1639 | 1810 | 1890 |
| 4 | 1312 | 1209 | 2038 | | |
| 5 | | 1418 | | | |
| 6 | | 1599 | | 3350 | 3400 |
| 7 | 1367 | 1741 | 2829 | 3720 | 3740 |
| Content | 1.80 | 6.13 | 5.24 | 7.12 | 6.64 |

Table 5 shows the evaluation results of the cumulative penetration amount (µg/cm²).

**[Table 6]**

| Day | Exelon^{®} patch | Example 1 | Example 2 | Example 3 | Example 4 |
|---|---|---|---|---|---|
| 0.5 | 40.2 | 29.9 | 31.5 | 27.9 | 29.7 |
| 1.5 | 11.4 | 15.7 | 17.6 | 26.6 | 29.6 |
| 2.5 | 1.3 | 14.1 | 19.2 | 20.8 | 22.0 |
| 3.5 | 1.7 | 13.5 | 16.7 | | 22.7 |
| 4.5 | | | | 21.4 | |
| 5.0 | | | | | |
| 5.5 | 0.8 | 11.6 | 11.0 | | |
| 6.5 | | | | 15.6 | 16.0 |
| Content | 1.80 | 6.13 | 5.24 | 7.12 | 6.64 |

Table 6 shows the results of Flux rate (µg/cm²/hour). From the experimental results obtained above, it was confirmed that the examples of the present invention can control the release of the drug continuously during the application period.

## Claims

1. A rivastigmine patch for transdermal treatment comprising a backing film; a rivastigmine matrix layer comprising rivastigmine or a pharmaceutically acceptable salt thereof; a self-adhesive matrix layer comprising an adhesive that adheres to the skin; and a release liner that is removed before use,
wherein the content per patch area of the rivastigmine or a pharmaceutically acceptable salt thereof is 2 to 15 mg/cm² based on the rivastigmine free base, and
the rivastigmine matrix layer comprises at least one selected from the group consisting of silicate, microcrystalline cellulose and crospovidone.

2. The rivastigmine patch of claim 1, wherein the content per patch area of the rivastigmine or a pharmaceutically acceptable salt thereof is 3.6 to 10.8 mg/cm2 based on the rivastigmine free base.

3. The rivastigmine patch of claim 2, wherein the content per patch area of the rivastigmine or a pharmaceutically acceptable salt thereof is 5.4 to 9 mg/cm² based on the rivastigmine free base.

4. The rivastigmine patch of claim 1, wherein the silicate is magnesium aluminometasilicate, calcium silicate or a mixture thereof.

5. The rivastigmine patch of claim 1, wherein the rivastigmine matrix layer comprises 10-50% by weight of rivastigmine, 10-30% by weight of microcrystalline cellulose, and the residual quantity of an acrylic adhesive based on the total weight of the rivastigmine matrix layer.

6. The rivastigmine patch of claim 1, wherein the rivastigmine matrix layer comprises 10-50% by weight of rivastigmine, 10-30% by weight of magnesium aluminometasilicate, and the residual quantity of an acrylic adhesive based on the total weight of the rivastigmine matrix layer.

7. The rivastigmine patch of claim 1, wherein the rivastigmine matrix layer comprises 10-50% by weight of rivastigmine, 5-25% by weight of crospovidone, and the residual quantity of an acrylic adhesive based on the total weight of the rivastigmine matrix layer.

8. The rivastigmine patch of claim 1, wherein the rivastigmine matrix layer comprises 10-50% by weight of rivastigmine, 2-15% by weight of calcium silicate, and the residual quantity of an acrylic adhesive based on the total weight of the rivastigmine matrix layer.

9. The rivastigmine patch of any one of claims 1 to 8, wherein the rivastigmine matrix layer of the patch further comprises an antioxidant.

10. The rivastigmine patch of any one of claims 1 to 8, wherein the rivastigmine patch comprises a release controlling membrane which is an ethylene vinyl acetate film between the rivastigmine matrix layer and the self-adhesive matrix layer.

11. The rivastigmine patch of claim 1, wherein the self-adhesive matrix layer comprises a mixture of two or more silicone adhesives having different properties.

12. The rivastigmine patch of claim 11, wherein the self-adhesive matrix layer comprises a mixture of BIO-PSA 7-4201 and 7-4301.

13. The rivastigmine patch of claim 12, wherein the self-adhesive matrix layer comprises a 1:1 weight ratio mixture of BIO-PSA 7-4201 and 7-4301.

## Patentansprüche

1. Rivastigmin-Patch zur transdermalen Behandlung, umfassend einen Trägerfilm; eine Rivastigmin-Matrixschicht, umfassend Rivastigmin oder ein pharmazeutisch annehmbares Salz davon; eine selbstklebende Matrixschicht, umfassend einen Klebstoff, der auf der Haut haftet; und ein Trennpapier, das vor der Benutzung entfernt wird,
wobei der Gehalt des Rivastigmins oder eines pharmazeutisch annehmbaren Salzes davon pro Patchfläche 2 bis 15 mg/cm², bezogen auf die freie Basis des Rivastigmins, beträgt und
die Rivastigmin-Matrixschicht zumindest eines ausgewählt aus der Gruppe bestehend aus Silikat, mikrokristalliner Cellulose und Crospovidon umfasst.

2. Rivastigmin-Patch nach Anspruch 1, wobei der Gehalt des Rivastigmins oder eines pharmazeutisch annehmbaren Salzes davon pro Patchfläche 3,6 bis 10,8 mg/cm2, bezogen auf die freie Basis des Rivastigmins, beträgt.

3. Rivastigmin-Patch nach Anspruch 2, wobei der Gehalt des Rivastigmins oder eines pharmazeutisch annehmbaren Salzes davon pro Patchfläche 5,4 bis 9 mg/cm², bezogen auf die freie Basis des Rivastigmins, beträgt.

4. Rivastigmin-Patch nach Anspruch 1, wobei es sich bei dem Silikat um Magnesiumaluminometasilikat, Calciumsilikat oder um eine Mischung davon handelt.

5. Rivastigmin-Patch nach Anspruch 1, wobei die Rivastigmin-Matrixschicht 10-50 Gew.-% Rivastigmin, 10-30 Gew.-% mikrokristalline Cellulose, und die restliche Menge eines Acrylklebstoffs, bezogen auf das Gesamtgewicht der Rivastigmin-Matrixschicht, umfasst.

6. Rivastigmin-Patch nach Anspruch 1, wobei die Rivastigmin-Matrixschicht 10-50 Gew.-% Rivastigmin, 10-30 Gew.-% Magnesiumaluminometasilikat, und die restliche Menge eines Acrylklebstoffs, bezogen auf das Gesamtgewicht der Rivastigmin-Matrixschicht, umfasst.

7. Rivastigmin-Patch nach Anspruch 1, wobei die Rivastigmin-Matrixschicht 10-50 Gew.-% Rivastigmin, 5-25 Gew.-% Crospovidon, und die restliche Menge eines Acrylklebstoffs, bezogen auf das Gesamtgewicht der Rivastigmin-Matrixschicht, umfasst.

8. Rivastigmin-Patch nach Anspruch 1, wobei die Rivastigmin-Matrixschicht 10-50 Gew.-% Rivastigmin, 2-15 Gew.-% Calciumsilikat, und die restliche Menge eines Acrylklebstoffs, bezogen auf das Gesamtgewicht der Rivastigmin-Matrixschicht, umfasst.

9. Rivastigmin-Patch nach einem der Ansprüche 1 bis 8, wobei die Rivastigmin-Matrixschicht des Patchs ferner ein Antioxidans umfasst.

10. Rivastigmin-Patch nach einem der Ansprüche 1 bis 8, wobei das Rivastigmin-Patch eine die Freisetzung kontrollierende Membran umfasst, bei der es sich um einen Ethylen-Vinylacetatfilm zwischen der Rivastigmin-Matrixschicht und der selbstklebenden Matrixschicht handelt.

11. Rivastigmin-Patch nach Anspruch 1, wobei die selbstklebende Matrixschicht eine Mischung von zwei oder mehreren Silikonklebstoffen umfasst, die unterschiedliche Eigenschaften aufweisen.

12. Rivastigmin-Patch nach Anspruch 11, wobei die selbstklebende Matrixschicht eine Mischung von BIO-PSA 7-4201 und 7-4301 umfasst.

13. Rivastigmin-Patch nach Anspruch 12, wobei die selbstklebende Matrixschicht ein Gewichtsverhältnis von 1:1 der Mischung von BIO-PSA 7-4201 und 7-4301 umfasst.

## Revendications

1. Patch de rivastigmine pour un traitement transdermique comprenant un film de support ; une couche de matrice de rivastigmine comprenant de la rivastigmine ou un sel pharmaceutiquement acceptable correspondant ; une couche de matrice auto-adhésive comprenant un adhésif qui adhère à la peau ; et une doublure de libération qui est éliminée avant utilisation,
la teneur par surface de patch de la rivastigmine ou d'un sel pharmaceutiquement acceptable correspondant étant de 2 à 15 mg/cm² sur la base de la base libre de la rivastigmine, et
la couche de matrice de rivastigmine comprenant au moins l'un choisi dans le groupe constitué par un silicate, une cellulose microcristalline et une crospovidone.

2. Patch de rivastigmine selon la revendication 1, la teneur par surface de patch de la rivastigmine ou d'un sel pharmaceutiquement acceptable correspondant étant de 3,6 à 10,8 mg/cm2 sur la base de la base libre de la rivastigmine.

3. Patch de rivastigmine selon la revendication 2, la teneur par surface de patch de la rivastigmine ou d'un sel pharmaceutiquement acceptable correspondant étant de 5,4 à 9 mg/cm² sur la base de la base libre de la rivastigmine.

4. Patch de rivastigmine selon la revendication 1, le silicate étant un aluminométasilicate de magnésium, un silicate de calcium ou un mélange correspondant.

5. Patch de rivastigmine selon la revendication 1, la couche de matrice de rivastigmine comprenant 10 à 50 % en poids de rivastigmine, 10 à 30 % en poids de cellulose microcristalline, et la quantité résiduelle d'un adhésif acrylique sur la base du poids total de la couche de matrice de rivastigmine.

6. Patch de rivastigmine selon la revendication 1, la couche de matrice de rivastigmine comprenant 10 à 50 % en poids de rivastigmine, 10 à 30 % en poids d'aluminométasilicate de magnésium, et la quantité résiduelle d'un adhésif acrylique sur la base du poids total de la couche de matrice de rivastigmine.

7. Patch de rivastigmine selon la revendication 1, la couche de matrice de rivastigmine comprenant 10 à 50 % en poids de rivastigmine, 5 à 25 % en poids de crospovidone, et la quantité résiduelle d'un adhésif acrylique sur la base du poids total de la couche de matrice de rivastigmine.

8. Patch de rivastigmine selon la revendication 1, la couche de matrice de rivastigmine comprenant 10 à 50 % en poids de rivastigmine, 2 à 15 % en poids de silicate de calcium, et la quantité résiduelle d'un adhésif acrylique sur la base du poids total de la couche de matrice de rivastigmine.

9. Patch de rivastigmine selon l'une quelconque des revendications 1 à 8, la couche de matrice de rivastigmine du patch comprenant en outre un antioxydant.

10. Patch de rivastigmine selon l'une quelconque des revendications 1 à 8, le patch de rivastigmine comprenant une membrane de libération contrôlée qui est un film d'éthylène acétate de vinyle entre la couche de matrice de rivastigmine et la couche de matrice auto-adhésive.

11. Patch de rivastigmine selon la revendication 1, la couche de matrice auto-adhésive comprenant un mélange de deux adhésifs de silicone ou plus ayant des propriétés différentes.

12. Patch de rivastigmine selon la revendication 11, la couche de matrice auto-adhésive comprenant un mélange de BIO-PSA 7-4201 et 7-4301.

13. Patch de rivastigmine selon la revendication 12, la couche de matrice auto-adhésive comprenant un rapport en poids 1 : 1 de BIO-PSA 7-4201 et 7-4301.
